# EUROPEAN PATENT SPECIFICATION

(11) **EP 3 042 639 B1**
(45) Date of publication and mention of the grant of the patent: **05.09.2018**
(21) Application number: 14840443.7
(22) Date of filing: 02.07.2014
(51) Int. Cl.: A61F 13/15, A61F 13/472, A61F 13/534, A61F 13/535, A61F 13/539

(54) **ABSORBENT PRODUCT**
SAUGFÄHIGES PRODUKT
PRODUIT ABSORBANT

(30) Priority: 02.09.2013 JP 2013181675
(43) Date of publication of application: 13.07.2016
(73) Proprietor: Kao Corporation, Chuo-Ku Tokyo 103-8210 (JP)
(72) Inventor: ISHIKAWA, Takehiro, Haga-gun Tochigi 321-3497 (JP); KIMURA, Mayumi, Haga-gun Tochigi 321-3497 (JP); OKU, Shogo, Haga-gun Tochigi 321-3497 (JP)
(74) Representative: Vossius & Partner Patentanwälte Rechtsanwälte mbB
(86) International application number: PCT/JP2014/067670
(87) International publication number: WO 2015/029587

(56) References cited:
- EP-A1- 2 538 908
- JP-A- 2003 093 441
- JP-A- 2006 081 558
- JP-A- 2007 097 954
- JP-A- 2008 529 721
- JP-A- 2010 142 367
- JP-A- 2012 125 365
- JP-A- 2013 111 127
- JP-A- 2013 154 017
- JP-U- H0 187 720
- US-A- 2 073 410
- US-A- 3 441 023
- US-A1- 2006 047 257
- US-A1- 2009 062 760

## Description

### Technical Field

The present invention relates to an absorbent article such as a sanitary napkin.

### Background Art

Known in the art is a technique that involves providing narrow slits or grooves in an absorbent member used in an absorbent article such as a sanitary napkin or a panty liner. For example, JP 2008-529721T discloses a method for forming three regions with different rigidities in an absorbent member, wherein one of the regions is formed by forming a plurality of long narrow holes in the absorbent member by removing the material forming the absorbent member. JP 2010-273842A discloses an absorbent article including an absorbent member that has a groove, wherein the opening width of the groove along a plane orthogonal to the thickness direction of the absorbent member decreases from one surface of the absorbent member toward the other surface thereof.

Also known in the art as an absorbent member used in an absorbent article is an absorbent member having a layered structure including two or more layers of absorbent sheets that are formed thinly by using adhesive means, such as a binder, with the aim of thinning down the absorbent member. Also known is a technique in which slits or openings are formed in the absorbent member having such a layered structure. For example, JP 2009-153835A discloses an absorbent article that uses an absorbent member in which: an upper absorbent sheet and a lower absorbent sheet are superposed; slits are provided in each absorbent sheet in the lateral direction; and the slits are arranged so as not to overlap one another in a planar view of the absorbent sheets.

### Summary of Invention

In the techniques disclosed in JP 2008 529721T and JP 2010 273842A, the inner surfaces of each slit or groove are made of absorbent fibers such as pulp. Thus, in the course of forming the slits or grooves, the absorbent fibers tend to be densified, which may make it difficult for liquid to diffuse to the interior of the absorbent member through the inner surfaces of the slits or grooves. This applies to the slits formed in the layered structure of absorbent sheets disclosed in JP 2009-153835A. On the inner surfaces of each slit, the layered absorbent sheets tend to be densified and integrated; this causes the adjacent absorbent sheets to be joined together, thus making it difficult for liquid to diffuse to the interior of the absorbent member.

The present invention provides an absorbent article including an absorbent assembly according to claim 1.

### Brief Description of Drawings

[Fig. 1] Fig. 1(a) is a plan view illustrating a skin-opposing surface side (topsheet side) of a sanitary napkin which is an embodiment of an absorbent article of the present invention, and Fig. 1(b) is a plan view illustrating only the absorbent member extracted from the sanitary napkin.
[Fig. 2] Fig. 2 is a schematic cross-sectional view taken along line II-II of Fig. 1.
[Fig. 3] Figs. 3(a) and 3(b) are partially enlarged plan views of absorbent members illustrating preferred arrangements of longitudinal slits in a central slit region.
[Fig. 4] Fig. 4 is a vertical cross-sectional view of a longitudinal slit in a central slit region.
[Fig. 5] Fig. 5 is a vertical cross-sectional view of a longitudinal slit in a side slit region.
[Fig. 6] Fig. 6 is a schematic cross-sectional view taken along line IV-IV of Fig. 1.
[Fig. 7] Fig. 7(a) is a partially enlarged plan view of a rear slit region in the sanitary napkin illustrated in Fig. 1, and Fig. 7(b) is a view of another embodiment corresponding to Fig. 7(a).
[Fig. 8] Fig. 8 is a schematic diagram illustrating steps for manufacturing the absorbent member in the sanitary napkin illustrated in Fig. 1.
[Fig. 9] Figs. 9(a) to 9(d) are schematic cross-sectional views of auxiliary absorbent members illustrating other examples of methods for folding up the absorbent sheet.

### Description of Embodiments

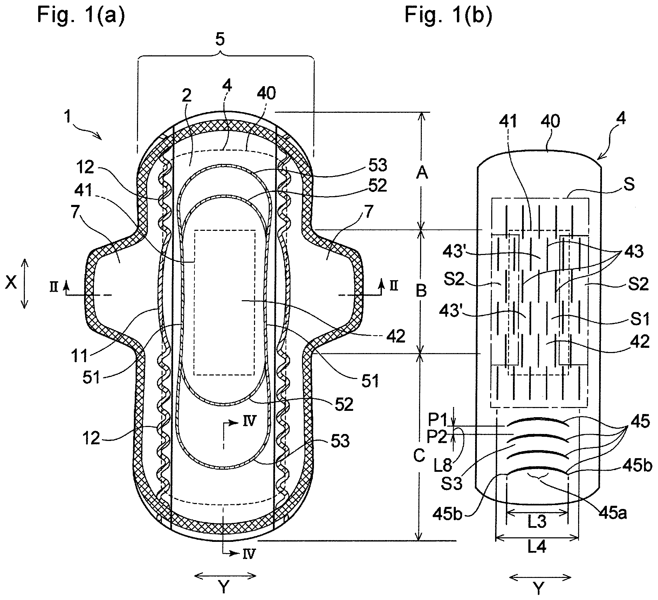

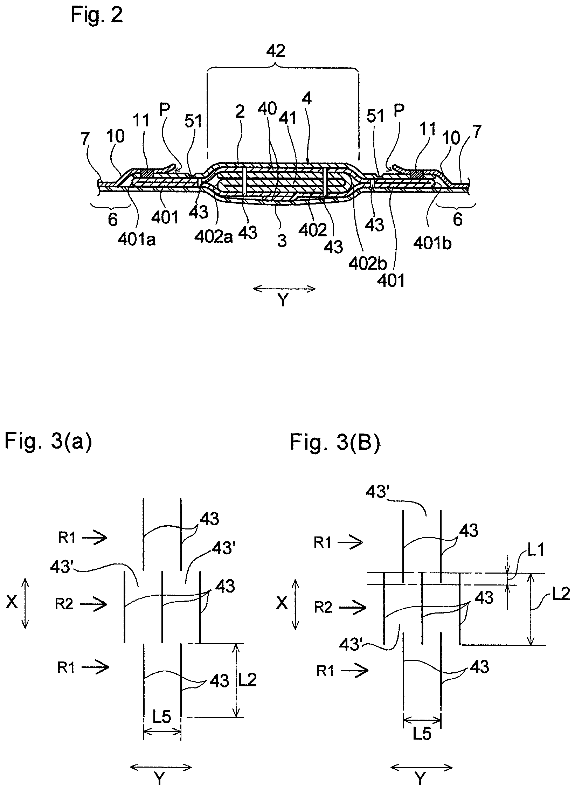

An absorbent article of the present invention is described below according to a sanitary napkin, which is a preferred embodiment of the invention, with reference to the drawings. As illustrated in Figs. 1 and 2, a napkin 1 of the present embodiment includes an absorbent assembly 5 that includes: a liquid-retentive absorbent member 4; a topsheet 2 arranged on the skin-opposing surface side of the absorbent member 4; and a backsheet 3 arranged on the non-skin-opposing surface side of the absorbent member 4. The napkin has a longitudinal direction X corresponding to the wearer's front/rear direction, and a lateral direction Y orthogonal to the longitudinal direction.

It should be noted that, in the present Description, the skin-opposing surface is the surface of the absorbent article, or its constituent members (such as the absorbent assembly 5), that faces the wearer's skin when the absorbent article is worn, and the non-skin-opposing surface is the surface of the absorbent article, or its constituent members, that faces the opposite side from the skin (i.e., the garment side) when the absorbent article is worn. The longitudinal direction X matches the length direction of the absorbent article (absorbent assembly), and the lateral direction Y matches the width direction (direction orthogonal to the length direction) of the absorbent article (absorbent assembly).

The absorbent assembly 5 has, in the longitudinal direction X: an excretion-section opposing region B arranged so as to oppose the wearer's fluid excretion section (e.g. the introitus) when the absorbent article is worn; a front region A arranged more toward the wearer's front side (stomach side) than the excretion-section opposing region B when the article is worn; and a rear region C arranged more toward the wearer's rear side (back side) than the excretion-section opposing region B when the absorbent article is worn.

In addition to the absorbent assembly 5, the napkin 1 includes a pair of wings 7, 7 extending outwardly in the lateral direction Y from the respective lateral sides of the absorbent assembly 5's excretion-section opposing region B that extend along the longitudinal direction X.

It should be noted that, in the absorbent article of the present invention, in cases where there are wings as in the napkin 1 of the present embodiment, the excretion-section opposing region is the region where the wings are provided in the longitudinal direction (the length direction of the absorbent article; the X direction in the figures) of the absorbent article (i.e., is the region sandwiched between each wing's joining base on one side in the longitudinal direction and the joining base on the other side). In an absorbent article without wings, the excretion-section opposing region is the region surrounded by a first fold line and a second fold line as counted from the absorbent article's front end in the longitudinal direction, the aforementioned first and second fold lines being the two fold lines (not illustrated) that traverse the absorbent article in the lateral direction (the width direction of the absorbent article; the Y direction in the figures) and that are created when the absorbent article is folded up in three into its individually-packaged form.

As illustrated in Fig. 2, the topsheet 2 covers the entire region of the skin-opposing surface of the absorbent member 4, and also extends outward in the lateral direction Y from the absorbent member 4's respective lateral side edges that extend along the longitudinal direction X. On the other hand, the backsheet 3 covers the entire region of the non-skin-opposing surface of the absorbent member 4, and also extends outward in the lateral direction Y from the absorbent member 4's respective lateral side edges that extend along the longitudinal direction X, and forms side flaps 6 together with later-described side sheets 10. The backsheet 3 and the side sheets 10 are joined together by a known joining means, such as an adhesive, heat sealing, ultrasonic sealing, etc., in extension portions extending from the absorbent member 4's respective lateral side edges which extend along the longitudinal direction X. The topsheet 2 and the absorbent member 4, as well as the backsheet 3 and the absorbent member 4, may be joined by an adhesive.

As illustrated in Fig. 2, the absorbent member 4 of the present embodiment has a layered structure which has base absorbent sheet 401 and a central absorbent sheet 402 and which has a plurality of layers, and includes a multi-layer section 42 in the excretion-section opposing region B, as illustrated in Figs. 1 and 2. As illustrated in Fig. 2, the multi-layer section 42 is a section where the number of layers of the absorbent sheets 401, 402 constituting the absorbent member 4 is greater than sections located in its periphery. The multi-layer section 42 in the present embodiment has a thickness that is thicker than that of sections located in its periphery, and forms a protruding section that protrudes toward the topsheet 2 side (the napkin 1's skin-opposing surface side) in the excretion-section opposing region B. Further, the absorbent member 4 includes: a main absorbent member 40 forming the outer shape of the absorbent member 4; and an auxiliary absorbent member 41 that is arranged so as to overlap a portion of the main absorbent member 40 and that is smaller than the main absorbent member 40. The auxiliary absorbent member 41 is located at least in the excretion-section opposing region B.

The absorbent member 4 of the present embodiment is preferably constituted only from the layered structure including the absorbent sheets 401, 402, and preferably, the absorbent member 4 does not include a fiber-stacked structure including absorbent fibers such as pulp. In the present invention, the absorbent sheet is a sheet-like absorbent structure made by forming an absorbent material, including pulp, into a thin sheet by using an adhesive means such as a binder.

The main absorbent member 40 consists of a structure in which the base absorbent sheet 401 including pulp is folded up. More specifically, the main absorbent member 40 is formed by inwardly folding the base absorbent sheet 401 at positions of fold lines 401a, 401b that extend along the longitudinal direction X. Stated differently, the base absorbent sheet 401 is in a state where both ends thereof are folded up (in a gate fold). At this time, the positions of the fold lines 401a, 401b are adjusted in such a manner that the inwardly-folded sections of the base absorbent sheet 401 partially overlap one another. Thus, the main absorbent member 40 has a layered structure including two layers and three layers consisting of the base absorbent sheet 401.

On the other hand, the auxiliary absorbent member 41 consists of a structure in which the central absorbent sheet 402 including pulp is folded up. More specifically, the auxiliary absorbent member 41 is made by: inwardly folding, along a fold line 402a that extends along the longitudinal direction X, a section of the central absorbent sheet 402 located outward of the fold line 402a; and then inwardly folding, along a fold line 402b, a section located outward of the fold line 402b toward the side of the section that has been folded first. The fold lines 402a, 402b are formed at positions that divide the central absorbent sheet 402's length in the lateral direction Y into three equal parts. Thus, the central absorbent sheet 402 is in a state where it is folded-up in a letter fold (tri-fold). Thus, the auxiliary absorbent member 41 has a layered structure including three layers consisting of the central absorbent sheet 402.

As illustrated in Fig. 2, the central absorbent sheet 402, which is in a state where it is folded-up in a letter fold (tri-fold), is arranged inside the base absorbent sheet 401, which is in a state where both ends thereof are folded up (in a gate fold). Stated differently, the base absorbent sheet 401 and the central absorbent sheet 402 have a nested folded-up structure. As a result, in the layered structure including the base absorbent sheet 401 and the central absorbent sheet 402, the base absorbent sheet 401 is layered on the central absorbent sheet 402 so as to extend outward from at least a portion--more specifically, the entire region--of the peripheral edge of the central absorbent sheet 402. It should be noted that, because the auxiliary absorbent member 41 is located in the excretion-section opposing region B as described above, the central absorbent sheet 402 constituting the auxiliary absorbent member 41 is also located in the excretion-section opposing region B.

The main absorbent member 40, which is made of a folded-up structure of the base absorbent sheet 401, has a substantially rectangular shape with rounded corners in a planar view, and extends from the front region A to the rear region C across the excretion-section opposing region B. On the other hand, the auxiliary absorbent member 41, which is made of a folded-up structure of the central absorbent sheet 402, has a substantially rectangular shape in a planar view, and is arranged from the excretion-section opposing region B to its vicinity in the rear region C. By arranging the auxiliary absorbent member 41 in a portion of the main absorbent member 40, the absorption capacity in a portion of the absorbent member 4 can be increased easily and efficiently. Instead of arranging the auxiliary absorbent member 41 inside the main absorbent member 40 made of the folded-up base absorbent sheet 401, the auxiliary absorbent member 41 may be layered on the skin-opposing surface side of the base absorbent sheet 401 constituting the main absorbent member 40, or may be layered on the non-skin-opposing surface side thereof.

As illustrated in Figs. 1 and 2, on the respective lateral sides extending along the longitudinal direction X on the absorbent assembly 5's skin-opposing surface (the topsheet 2's skin-opposing surface), a pair of side sheets 10, 10 is arranged over substantially the entire length of the absorbent assembly 5 in the longitudinal direction X so as to overlap the absorbent member 4's respective lateral sides, which extend along the longitudinal direction X, in a planar view. Each of the side sheets 10, 10 is joined to the absorbent assembly 5 (topsheet 2) by: a linear first joining line 11 located in the excretion-section opposing region B; and linear second joining lines 12 located on the front and rear, in the longitudinal direction X, of the first joining line 11 (i.e., in the front region A and rear region C). The first joining line 11 is a curved line that protrudes outward in the lateral direction Y in a planar view. The second joining line 12 is a line (zigzag line) that extends so as to intersect with the longitudinal direction in a planar view. The side sheet 10 is made of a water-repellent nonwoven fabric. By joining each side sheet 10 onto the absorbent assembly 5's skin-opposing surface (the topsheet 2) by the joining lines 11, 12, a space P defined by the side sheet 10 and the topsheet 2 is formed on a side more inward in the lateral direction Y than the joining lines 11, 12, as illustrated in Fig. 2. Each space P is opened toward the absorbent assembly 5's center in the lateral direction Y; thus, excreted fluid, such as menstrual blood, that flows outward from the center in the lateral direction Y is captured by the spaces P, and as a result, leakage of excreted fluid is prevented effectively.

As illustrated in Fig. 1, the side flaps 6 project greatly outward in the lateral direction Y in the excretion-section opposing region B. Thus, a pair of wings 7, 7 extends on the absorbent assembly 5's respective lateral sides that extend along the longitudinal direction X. Further, as illustrated in Fig. 1, the topsheet 2 and the backsheet 3 extend outward in the longitudinal direction X from the absorbent member 4's front end and rear end in the longitudinal direction X, and their extension portions are joined together by a known joining means--such as an adhesive, heat sealing, or ultrasonic sealing--to form end sealed portions.

The wings 7 are used by being folded toward the non-skin-opposing surface side in the crotch section of a garment, such as underpants. As illustrated in Fig. 1, in a planar view, each wing 7 has a substantially trapezoidal shape in which the lower base (the side longer than the upper base) is located on the side of the absorbent assembly 5's lateral side. A wing adhesive section (not illustrated) for fixing the wing 7 (napkin 1) onto a garment such as underpants (not illustrated) is formed on the non-skin-opposing surface of each wing 7. With the wing adhesive section, each wing 7--which is folded back toward the non-skin-opposing surface (outer surface) in the garment's crotch section when the napkin is in use--can be attached and fixed to the crotch section. Further, an assembly adhesive section (not illustrated) for fixing the absorbent assembly 5 onto a garment such as underpants is formed on the non-skin-opposing surface of the absorbent assembly 5. It should be noted that, in the napkin 1, the borderline between the absorbent assembly 5 and each wing 7 is a straight line (not illustrated) connecting both joining bases, in the longitudinal direction X, of the wing 7.

Although not illustrated, it is preferable that the napkin 1 further includes a second sheet provided between the absorbent member 4 and the topsheet 2. Preferably, the second sheet is a liquid-permeable fiber sheet in which the constituent fibers are synthetic fibers. Particularly, by constituting the second sheet from fibers obtained by subjecting hydrophobic synthetic fibers to a hydrophilic treatment, liquid diffusibility can be kept low. Further, from the viewpoint of mechanical compatibility as well as reduced diffusibility, it is preferable that the synthetic fiber constituting the second sheet is a weakly hydrophilic fiber. The second sheet may be formed by using fibers made of a resin--such as polyethylene, polypropylene, a polyester such as polyethylene terephthalate or polybutylene terephthalate, or a polyamide such as nylon-6 or nylon-66--singly, or by mixing two or more types of the fibers. Herein, "mixing" encompasses the use of making two or more types of resins having different melting points into a core-sheath-type conjugate fiber or a side-by-side conjugate fiber.

In order to make the second sheet into a sheet having high permeability and moderate diffusibility, the thickness is preferably 0.15 mm or greater, more preferably 0.2 mm or greater, and preferably 0.4 mm or less, more preferably 0.3 mm or less. In consideration of the state in which the diaper is worn, the thickness is measured under a load of 0.5 kPa.

As for the second sheet, it is possible to preferably use an air-through nonwoven fabric in which the intersecting points between intersecting synthetic fibers are joined by fusion-bonding according to an air-through method. It should be noted that, other than air-through nonwoven fabrics, it is possible to use, for example, point-bonded nonwoven fabrics, spun-bonded nonwoven fabrics, or spun-laced nonwoven fabrics. In cases where the second sheet is an air-through nonwoven fabric, the basis weight is preferably from 15 g/m² to 40 g/m² inclusive.

It is preferable that the topsheet 2 and the second sheet are partially fixed. In this way, the topsheet 2 can be prevented from rising up, and also, the adhesion between the topsheet 2 and the second sheet is improved and liquid can be transferred more easily. For the same reasons, it is preferable that the second sheet and the absorbent member 4 are partially fixed. Here, "partially fixed" means that there are fixing portions consisting of dots or lines, and, although the fixing portions do not have to be distributed evenly, the fixing portions are distributed over the entire sheet surface, and the sheets to be fixed are not fixed over the entire surface. The area of the fixing portions is preferably from about 10% to 70% inclusive with respect to the entire area of the sheet to be fixed. More specifically, the topsheet 2 and the second sheet are partially fixed by intermittently applying an adhesive or by intermittently applying heat sealing. In cases of intermittently applying an adhesive, the topsheet 2 and the second sheet are partially bonded in a state where body fluid permeability is maintained between the topsheet 2 and the second sheet by employing known means, for example, by intermittently applying an adhesive with a slot-coating gun, by spirally applying an adhesive with a spiral spray gun, by intermittently applying an adhesive in an atomized form with a spray gun, or by applying an adhesive in dots with a dot gun. Examples of applicable adhesives that may be preferably used include hot-melt adhesives.

Examples of hot-melt adhesives include styrene-based and olefin-based adhesives. Examples of styrene-based hot-melt adhesives that may be used include styrene-butadiene-styrene (SBS) copolymers, styrene-isoprene-styrene (SIS) copolymers, styrene-ethylene-butylene-styrene (SEBS) copolymers which are hydrogenated products of SBS, and blended hot-melt adhesives in which two or more types of the above are blended. Particularly, a blended hot-melt adhesive including SIS and SBS or a blended hot-melt adhesive including SIS and SEBS is preferably used in the present invention because of the ease of balancing tack force and cohesive force. The amount of hot-melt adhesive applied is preferably from 3 g/m² to 10 g/m² inclusive.

In cases of intermittently applying heat sealing, the topsheet 2 and the second sheet are partially fusion-bonded by, for example, employing a heat-embossing roller having a plurality of projections and a flat roller, transmitting a layered sheet consisting of the topsheet 2 and the second sheet between the heat-embossing roller and the flat roller, and embossing the layered sheet. The distance between the plurality of fusion-bonded points formed by fusion-bonding is preferably from 5 mm to 15 mm inclusive from the viewpoint of maintaining body fluid permeability while achieving moderate adhesion between the topsheet 2 and the second sheet.

As illustrated in Figs. 1 and 2, linear grooves, which are formed by recessing the topsheet 2 and the absorbent member 4 in an integrated fashion toward the backsheet 3 side, are formed in the absorbent assembly 5's skin-opposing surface (the topsheet 2's skin-opposing surface). The linear grooves include: longitudinal grooves 51 that extend along the longitudinal direction X in the excretion-section opposing region B; and first and second lateral grooves 52, 53 formed so as to extend along the lateral direction Y in the front region A and the rear region C. The linear grooves can be formed according to an ordinary method, such as compression involving or not involving heat, or embossing such as ultrasonic embossing. Although not illustrated, in the linear grooves, the topsheet 2 and the absorbent member 4 are compression-bonded by an adhesive, or are integrated by thermal fusion-bonding etc.

With these linear grooves, it is possible to suppress liquid diffusion in the planar direction of the absorbent member 4, and effectively prevent liquid leakage from the periphery of the napkin 1. The width (length in the direction orthogonal to the length direction) of the linear groove and its depth can be set similarly to linear grooves employed in this type of absorbent article. Further, as regards the term "linear" as used in the linear grooves, the shape of the groove (recess) is not limited to a straight line in a planar view, but includes curved lines, and the lines may be continuous lines or broken lines. For example, the linear groove may consist of a row of a multitude of discontinuous embossed dots. It should be noted that, as illustrated in Fig. 1, the napkin 1 of the present embodiment is formed so as to have left-right symmetry with respect to a center line (not illustrated) that divides the absorbent assembly 5 in two in the lateral direction Y.

As illustrated in Figs. 1 and 3, in the napkin 1 of the present embodiment, the absorbent member 4 includes, in the excretion-section opposing region B, an excretion-section slit region S in which a plurality of longitudinal slits 43 extending along the longitudinal direction X are formed in a dispersed state. The excretion-section slit region S includes a central slit region S1 and side slit regions S2. The central slit region S1 is located in a central section, in the lateral direction Y, of the absorbent member 4 in the excretion-section opposing region B. The central slit region S1 substantially matches the multi-layer section 42, i.e., the section where the base absorbent sheet 401 and the central absorbent sheet 402 are layered. The side slit regions S2 are located outward, in the lateral direction Y, of the central slit region S1. The side slit regions S2 substantially match the sections in which the absorbent member 4 is formed only of the base absorbent sheet 401.

The longitudinal slits 43 in the excretion-section slit region S are formed in a dispersed state in both the longitudinal direction X and the lateral direction Y. As illustrated in Fig. 2, each longitudinal slit 43 is formed by cutting the absorbent member 4 toward the thickness direction thereof, and preferably penetrates the layered structure including the absorbent sheets 401, 402 constituting the absorbent member 4. Preferably, the longitudinal slits 43 are not formed in the topsheet 2 and the backsheet 3, which are respectively arranged on the upper side and the lower side of the absorbent member 4, and are not formed in a nonwoven fabric-made intermediate sheet that may be arranged between the topsheet 2 and the absorbent member 4.

The longitudinal slits 43 are formed separately and independently from one another, and there is a non-slit section 43' located between adjacent longitudinal slits 43. The entire region of the periphery of each longitudinal slit 43 is surrounded by the non-slit section 43'.

As described above, the longitudinal slit 43 is formed by cutting the absorbent member 4 along the thickness direction thereof, and is constituted by a cut that substantially has no width, or is a narrow space with a width that is preferably 1.5 mm or less. In cases where the longitudinal slit 43 has a width, the width refers to the length of the longitudinal slit 43 in the direction orthogonal to the direction along which the longitudinal slit 43 extends.

As illustrated in Figs. 3(a) and 3(b), in the absorbent member 4, a plurality of slit rows R1, R2--each including a plurality of the longitudinal slits 43 separated from one another in the lateral direction Y--are formed in the longitudinal direction X of the napkin 1. Figs. 3(a) and 3(b) illustrate examples in which slit rows R1--each including two longitudinal slits 43 separated from one another in the lateral direction Y--and slit rows R2--each including three longitudinal slits 43 separated from one another in the lateral direction Y--are formed alternately in the longitudinal direction X. However, the excretion-section slit region S only needs to include at least two slit rows arranged in the longitudinal direction X, each slit row including two longitudinal slits 43 separated from one another in the lateral direction Y. It is, however, preferable to form three or more slit rows, more preferably four or more slit rows, and even more preferably five or more slit rows, in the longitudinal direction X. Further, as for the number of the longitudinal slits included in each slit row and separated from one another in the lateral direction Y, preferably two or more longitudinal slits, more preferably three or more longitudinal slits, and even more preferably four or more longitudinal slits, are included in each slit row.

It is preferable that there is no space between the slit rows adjacent to one another in the longitudinal direction X of the napkin 1. The expression "there is no space P between adjacent slit rows" encompasses: cases where the positions of the respective end sections of slits 43 in adjacent slit rows match one another, as illustrated in Fig. 3(a); and cases where adjacent slit rows partially overlap one another in the longitudinal direction X, as illustrated in Fig. 3(b). In cases of arranging two slit rows in the longitudinal direction X in a partially overlapping state as illustrated in Fig. 3(b), the overlapping length L1 is preferably 20% or less, more preferably 10% or less, of the length L2, in the direction X, of each longitudinal slit constituting the slit rows.

As illustrated in Figs. 3(a) and 3(b), the positions of the slits in the slit rows adjacent to one another in the longitudinal direction X of the napkin 1 are misaligned in the lateral direction Y of the napkin 1. More specifically, as regards the longitudinal slits 43 in the two slit rows R1, R2 adjacent to one another in the longitudinal direction X, it is preferable that each slit 43 in one slit row R1 is located in the central section between two slits 43 in the other slit row R2; however, the slit 43 in one slit row R1 may be formed so as to be located closer to either one of the two slits 43 in the other slit row R2.

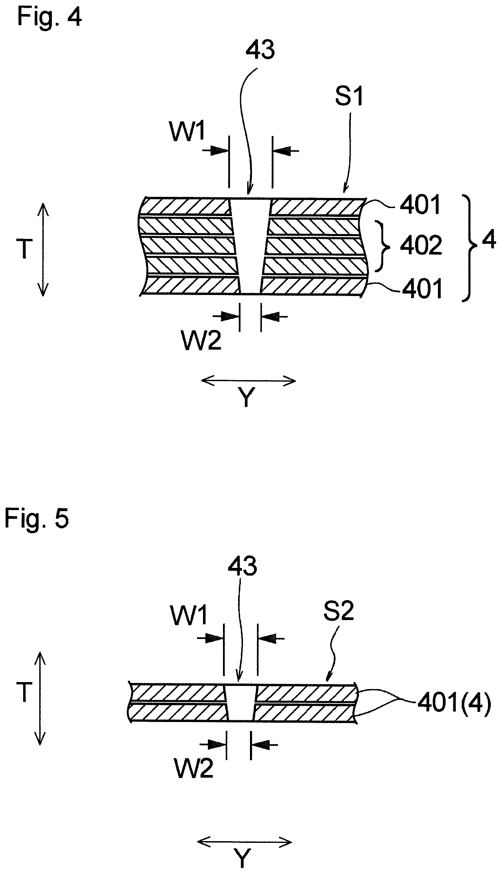

Fig. 4 illustrates a vertical cross-sectional view of a longitudinal slit 43 formed in the central slit region S1, and the absorbent member in sections in the vicinity of the longitudinal slit. Fig. 5 is a vertical cross-sectional view of a longitudinal slit 43 formed in the side slit region S2, and the absorbent member in sections in the vicinity of the longitudinal slit. In the central slit region S1 illustrated in Fig. 4, the longitudinal slit 43 is formed in a layered structure having a five-layer structure including: a three-layer structure part formed by folding up the central absorbent sheet 402; and the layers 401, 401 of the base absorbent sheet located on the upper side and lower side of the three-layer structure part. The longitudinal slit 43 penetrates the five-layered structure over the entire region in the thickness direction thereof. On the other hand, in the side slit region S2 illustrated in Fig. 5, the longitudinal slit 43 is formed in a layered structure having two layers made by folding up the base absorbent sheet 401. The longitudinal slit 43 penetrates the two-layered structure over the entire region in the thickness direction thereof.

As illustrated in Fig. 4, in the five-layered structure constituting the central slit region S1, a portion of the plurality of layers of the absorbent sheets forming the layered structure is in a non-joined state in a section where each longitudinal slit 43 is formed. The expression "the absorbent sheets are in a non-joined state" means that the absorbent sheets adjacent to one another in the thickness direction are not joined by any of various types of joining means such as compression-bonding, adhesion, fusion-bonding, etc. Fig. 4 illustrates a state in which all of the absorbent sheets adjacent to one another in the thickness direction are in a non-joined state. In this figure, a void is provided between adjacent absorbent sheets with the aim of visually illustrating that the absorbent sheets are in a non-joined state (likewise for Fig. 5). However, in an actual layered structure, the void may or may not be formed. It is preferable to form a void between adjacent absorbent sheets from the viewpoint of increasing liquid retention capacity at the time of repeatedly absorbing liquid and to prevent a reduction in absorption speed. Further, in Fig. 4, all of the absorbent sheets adjacent to one another in the thickness direction are in a non-joined state, but it will suffice if the non-joined state between absorbent sheets is present in at least one section of the adjacent absorbent sheets.

Macroscopically, the absorbent sheet has a flat shape, but microscopically, the surface of the absorbent sheet has fine projections and depressions. Thus, voids are created between the absorbent sheets simply by superposing the absorbent sheets. These voids are maintained so long as the layered structure of the absorbent sheets is not intentionally compressed.

Because the absorbent sheets adjacent to one another in the thickness direction are in a non-joined state in a section where each longitudinal slit 43 is formed, when fluid excreted to the napkin 1 reaches the absorbent member 4 through the topsheet 2 and flows into the longitudinal slits 43, the liquid diffuses smoothly in the planar direction of the absorbent member 4 through the inner surfaces of each longitudinal slit 43. Particularly, between absorbent sheets adjacent to one another in the thickness direction, liquid is easily drawn in by capillary action, and thus, liquid is diffused in the planar direction even more easily. As a result, liquid absorption speed is increased.

Also in the side slit regions S2, as in the central slit region S1, a section, in which each longitudinal slit 43 is formed, of each side slit region S2 has the two-layered structure, which constitutes each side slit region S2, of the absorbent sheets which is in a non-joined state. Thus, also in the side slit regions S2, liquid absorption speed is increased according to the same mechanism as in the central slit region S1.

As illustrated in Figs. 4 and 5, when the longitudinal slit 43's length in the lateral direction Y is viewed along the thickness direction T of the absorbent member 4, the slit's length W1 on the topsheet side is longer than the slit's length W2 on the backsheet side. In Figs. 4 and 5, the longitudinal slit 43's length in the lateral direction Y is illustrated so as to gradually decrease continuously from the topsheet side to the backsheet side. However, the longitudinal slit 43's length in the lateral direction Y does not have to change in this manner, and for example, the length may decrease stepwise from the topsheet side to the backsheet side. By making the longitudinal slit 43's length W1 on the topsheet side longer than the length W2 on the backsheet side, liquid can be easily taken into each longitudinal slit 43, and liquid absorption speed is increased also by this configuration. Also, because each longitudinal slit 43 has this structure, external force applied to the napkin 1 is less likely to be transmitted to the backsheet side of the absorbent member 4 in a state where the napkin 1 is worn, and thus, the napkin 1 can be fixed stably to underwear. Because external force is less likely to be transmitted to the backsheet side of the absorbent member 4, the flat shape on the backsheet side of the absorbent member 4 can be maintained, and this also allows the napkin 1 to be fixed stably to underwear. As a result, the napkin 1 is less likely to move out of place from its proper attachment position even when external force is applied to the napkin 1, and thus, reduction in absorptivity and degradation in comfortableness when worn are prevented effectively.

The cross-sectional shape of the longitudinal slit 43 along the thickness direction T of the absorbent member 4 can be observed at a magnification of 20x to 100x with a microscope (e.g. VHX-1000 from Keyence Corporation). A cross section along the thickness direction T of the absorbent member 4 can be formed, for example, by cutting the absorbent member 4 with a single-edged razor from Feather Safety Razor Co., Ltd. The ratio W1/W2 between the aforementioned lengths W1 and W2 is preferably 5 or greater, more preferably 10 or greater. Also, W1/W2 is preferably 80 or less, more preferably 60 or less. For example, W1/W2 is preferably from 5 to 80 inclusive, more preferably from 10 to 60 inclusive.

In a section where the longitudinal slit 43 is formed, it is preferable that the density of the absorbent sheets in each longitudinal slit's vicinity is higher than the density of the absorbent sheets in the aforementioned non-slit section 43'. In this way, liquid that has flowed into the longitudinal slit 43 can be easily drawn in between the absorbent sheets more quickly by capillary action. From the viewpoint of further enhancing this effect, the density of the absorbent sheets in each longitudinal slit 43's vicinity is preferably at least 1.05 times, more preferably at least 1.2 times, and preferably at most twice, more preferably at most 1.8 times, the density of the absorbent sheets in the non-slit section 43'. For example, the density of the absorbent sheets in each longitudinal slit 43's vicinity is preferably from 1.05 times to twice, more preferably from 1.2 times to 1.8 times, the density of the absorbent sheets in the non-slit section 43'.

The aforementioned density can be measured as follows. The density of the absorbent sheets in the longitudinal slit's vicinity, as well as in the non-slit section 43', was measured by: cutting out the absorbent sheets in the longitudinal slit's vicinity, as well as in the non-slit section 43', into a 3-mm-wide, 20-mm-long test specimen; measuring the thickness in the widthwise central section of the test specimen with a non-contact laser displacement meter (from Keyence Corporation; displacement meter: LK-GD500; laser head: LK-G30); measuring the mass of the test specimen with an electronic balance scale (electronic balance scale GR-300 from A&D Co., Ltd., with a precision of up to the ten-thousandths place); calculating the volume from the measured thickness; and dividing the mass by the volume.

Returning to Fig. 1, the absorbent member 4 includes, in the rear region C located more toward the rear than the excretion-section opposing region B, a plurality of rear slits 45 each extending so as to have its length direction along a direction intersecting with the longitudinal direction X of the napkin 1, as illustrated in the figure. As illustrated in Figs. 1(b), 6, and 7(a), each rear slit 45 has a shape, in a planar view, in which the position of the slit 45's central section 45a in its length direction is located more toward the front region A than the positions of both end sections 45b, 45b in its length direction, and more preferably, the rear slit 45 has a shape, in a planar view, that is curved protrudingly toward the front region A. The plurality of rear slits 45 are formed with an interval therebetween in the longitudinal direction X of the absorbent article.

Further, as illustrated in Fig. 1(b), the length L3, in the lateral direction Y, of a rear slit region S3 formed by the rear slits 45 is shorter than the length L4, in the direction Y, of the excretion-section slit region S. In Fig. 1(b), for convenience of visibility, the excretion-section slit region S is illustrated with a slightly wider area--both in the longitudinal direction X and the lateral direction Y--than the actual area in which the longitudinal slits 43 are distributed. However, the excretion-section slit region S in the present invention is the area in which the longitudinal slits 43 are distributed, and the length L4 thereof in the lateral direction Y is the distance between the outer edge of the respective longitudinal slits 43 located at opposite ends in the lateral direction Y, as illustrated in Fig. 1(b).

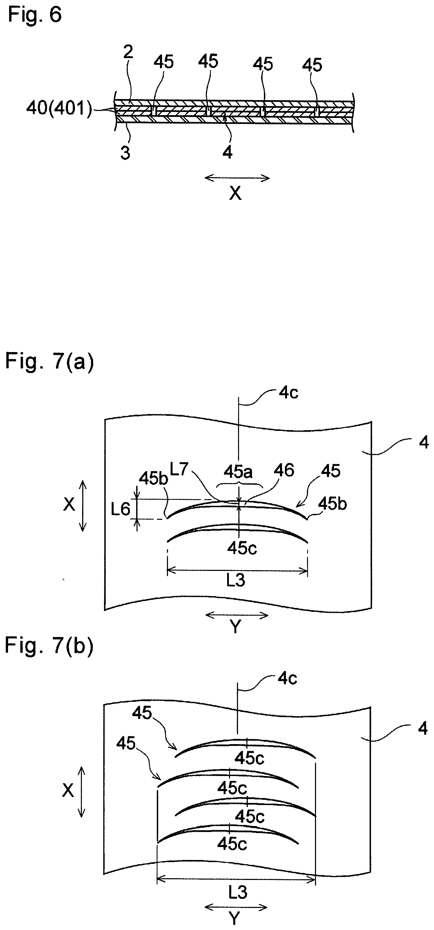

Further, the rear slit region S3 formed by the rear slits 45 is a region inside the smallest rectangle among various rectangles having two sides along the longitudinal direction X and two sides along the lateral direction Y and surrounding the entirety of the rear slits 45 (all of the rear slits in cases where there are a plurality of slits). In Fig. 1(b), the rear slit region S3 is also illustrated with a slightly wider area for convenience of visibility etc. In cases where a plurality of rear slits 45 are formed in the longitudinal direction X with their positions staggered in the lateral direction Y as illustrated in Fig. 7(b), the length L3 of the rear slit region S3 in the lateral direction Y is the distance between the two outermost end sections.

As illustrated in Fig. 7(a), in the napkin 1 of the present embodiment, the center position 4c that bisects the absorbent member 4's length in the lateral direction substantially matches the center position 45c that bisects the rear slit 45's length in the length direction. Here, the expression "substantially match" means that the center position 4c of the absorbent member 4 may be misaligned from the center position 45c of the rear slit by a length that is 10% of the absorbent member 4's length in the lateral direction.

The length of each of the rear slits 45, 45 in the length direction is substantially the same. Here, the expression "substantially the same" means that the difference in length is within 5% of the length L3 of the rear slit region S3 in the lateral direction Y. Further, the center position 45c of each of the rear slits 45, 45 substantially matches one another. Here, the expression "substantially match" means that the center positions may be misaligned from one another by a length that is 5% of the absorbent member 4's length in the lateral direction.

In the rear slit region S3, it is preferable that the two layers of the base absorbent sheet 401 forming the layered structure are in a non-joined state in a section where each rear slit 45 is formed, as with the longitudinal slits 43 formed in the aforementioned excretion-section slit region S. In this way, liquid absorption speed is increased also in the rear region C, and not only in the excretion-section opposing region B. From the viewpoint of further increasing liquid absorption speed, it is preferable that, when the rear slit 45's length in the longitudinal direction X is viewed along the thickness direction of the absorbent member 4, the rear slit 45's length on the topsheet side is longer than the length on the backsheet side. From the same viewpoint, it is preferable that the density of the base absorbent sheet 401 in each rear slit 45's vicinity is higher than the density of the base absorbent sheet 401 in a non-slit section located between the rear slits 45. The length of the rear slit 45 in the longitudinal direction X and the density in the vicinity of the rear slit 45 can be measured according to the same methods as in the case of the aforementioned longitudinal slits 43.

According to the napkin 1 of the present embodiment, it is possible to achieve the aforementioned advantageous effect that liquid absorption speed is increased, and in addition, achieve an advantageous effect that deep folding creases are less likely to be created in the absorbent member 4 and the napkin 1 because the absorbent member 4 deforms finely at the various sections in the excretion-section slit region, even if a force that compresses the absorbent member 4 in the lateral direction Y is applied by the wearer's thighs when the napkin 1 is worn. This prevents such deep folding creases from causing an uncomfortable feel to the wearer, and also prevents liquid from flowing along the folding creases and leaking. Particularly, in the napkin 1 of the present embodiment, there is no space between adjacent slit rows R1, R2, and thus, the napkin 1 is less likely to get twisted due to pressure from the thighs, compared to cases where there is a space between the slit rows R1, R2. In cases where there is a space between adjacent slit rows R1, R2, sections in the slit rows R1, R2 can deform so as to conform to movement and body shape and deep folding creases are less likely to be created, but folding creases are created in sections between the slit rows when the napkin is worn, and those folding creases are likely to propagate to slit-less sections within the slit rows, thus causing twisting of the napkin. Furthermore, a difference in rigidity arises between the sections where the slit rows are provided and the section in the space between the slit rows, and this may cause the napkin to be folded at the boundary therebetween and may impair fittability to the body.

Moreover, in the napkin 1 of the present embodiment, a plurality of rear slits 45 that extend along a direction intersecting with the longitudinal direction X of the absorbent article are formed in the rear region C, the rear slit 45 having a shape, in a planar view, that is curved protrudingly toward the front region A. Thus, even in sections that extend from the vicinity of the rear end section of the excretion-section opposing region B up to the rear region C and that need to be curved along the front/rear direction so as to conform to the body shape while receiving force from the thighs, the absorbent member 4 and the napkin 1 easily deform so as to conform to the wearer's movement and body shape. Thus, the napkin has excellent fittability and leakage preventability and is also comfortable to wear, even though an absorbent member 4 consisting of a laminate of absorbent sheets is used.

Further, because the length L3 of the rear slit region S3 is equal to or shorter than the length L4 of the excretion-section slit region S (L3≤L4), it is possible to achieve advantageous effects that the napkin can be prevented from excessively wedging into the gluteal cleft and the napkin can be prevented from getting twisted in the rear region C. The excretion region is the region that receives the greatest pressure from the thighs, and thus, it is preferable to form slits over the absorbent member's entire width to the extent possible, in order to make the napkin conform to the body's complicated shape and movement. However, the rear region hardly receives any pressure from the thighs; rather, it is more important to make the napkin conform to the body along its front/rear direction. Forming slits in a direction intersecting with the excretion-section opposing region B can prevent creases and twists--which are created along the napkin 1's front/rear direction in the excretion region by the pressure from the thighs--from propagating to the rear region C; and by making the width of the rear slit equal to or narrower than the width of the slit region in the excretion-section opposing region B, the rear region C can conform to the body without becoming too soft, and can deform so as to envelop the buttocks. By designing the length L3 of the rear slit region S3 to have the same length as the length L4 of the excretion-section slit region S, it is possible to further enhance the effect of preventing the propagation, to the rear region C, of creases and twists created along the napkin 1's front/rear direction in the excretion-section opposing region B. Further, the length of each of the rear slits 45 in the length direction may be gradually shortened toward the rear of the napkin 1, or only the rearmost rear slit 45 may be made shorter than the other rear slits 45 arranged more toward the front. It should be noted that, in cases where the lengths of the plurality of rear slits 45 are different from one another and their center positions, in the length direction, are aligned, the length L3 of the rear slit region S3 matches the straight-line distance between both ends, in the length direction, of the longest rear slit 45. In cases where the length of each of the rear slits 45 is gradually shortened toward the rear, it is possible to effectively prevent the napkin from wedging into the gluteal cleft while allowing the napkin to conform, to a certain extent, to the body's curved shape in the front/rear direction. Further, by designing the length of the rearmost rear slit 45 to be short, it is possible to prevent uncomfortableness caused by creases and folds which are created in slit-less sections located more toward the rear than the rear slits 45 and created as a result of a difference in rigidity between the slit region and the slit-less region. It is more effective particularly if the rearmost slit and the end section of the absorbent member 4 are located relatively near one another. It is further preferable if the length L3 of the rear slit region S3 is from 50% to 100% inclusive of the length L4 of the excretion-section slit region S.

In cases where the napkin manufacturing process involves a step of transporting the absorbent member, or a continuous absorbent member, along its length direction and meanwhile forming rear slits that extend along a direction orthogonal to the transporting direction, the absorbent member etc. is prone to tear when the absorbent member etc. after the formation of the slits is pulled in the transporting direction. In contrast, by forming each rear slit 45 in a shape in which the slit's central section 45a in its length direction is located more toward the front region than the positions of both end sections 45b, 45b in its length direction, the overlapping length between the rear slit 45 and a straight line extending along the direction orthogonal to the transporting direction can be reduced, and thus, the absorbent member etc. can be reliably prevented from tearing. From the viewpoint of workability, the ease of forming a gap (described below), and the prevention of tearing of the absorbent member etc. during transportation, it is preferable that the rear slit 45 has a shape, in a planar view, that is curved protrudingly toward the front region, and for example, has an arc shape, a parabolic shape, or a U-shape.

From the viewpoint of achieving the aforementioned effects even more reliably, it is preferable that the absorbent member 4 has one or more of the following features. The longitudinal slit 43's length L2 (cf. Fig. 3) along the longitudinal direction X in the excretion-section slit region S is preferably 10 mm or greater, more preferably 15 mm or greater, and preferably 35 mm or less, more preferably 30 mm or less, and preferably from 10 mm to 35 mm inclusive, more preferably from 15 mm to 30 mm inclusive.

The distance L5 (cf. Fig. 3) between the longitudinal slits 43 within the same slit row in the excretion-section slit region S is preferably 3 mm or greater, more preferably 7 mm or greater, and preferably 20 mm or less, more preferably 15 mm or less, and preferably from 3 mm to 20 mm inclusive, more preferably from 7 mm to 15 mm inclusive.

In a region with the same length, in the longitudinal direction, as the excretion-section slit region S, an area from each end section of the absorbent member 4 to a point that is preferably at least 5 mm, more preferably at least 7.5 mm, inward from the end section preferably constitutes a non-slit section wherein no longitudinal slit 43 is formed. Further, an area preferably up to 15 mm, more preferably up to 12.5 mm, preferably constitutes a non-slit section. For example, it is preferable that an area from each end section to a point that is from 5 mm to 15 mm, more preferably from 7.5 mm to 12.5 mm, from the end section constitutes a non-slit section wherein no longitudinal slit 43 is formed. Here, the "end section" of the absorbent member 4 refers to the absorbent member 4's lateral side edge extending along the longitudinal direction X.

The length L4 of the excretion-section slit region S in the lateral direction Y is preferably 30% or greater, more preferably 40% or greater, and preferably 85% or less, more preferably 75% or less, of the absorbent member 4's length in the direction Y in the excretion-section opposing region B. The length L4 of the excretion-section slit region S in the lateral direction Y is preferably 30 mm or greater, more preferably 40 mm or greater, and preferably 55 mm or less, more preferably 45 mm or less.

The length L3, in the lateral direction Y, of the rear slit region S3 formed by the rear slits 45 is preferably 30% or greater, more preferably 40% or greater, and preferably 65% or less, more preferably 55% or less, of the absorbent member 4's length in the same direction Y in the section where the rear slits 45 are provided.

The ratio of the length L3, in the lateral direction Y, of the rear slit region S3 formed by the rear slits 45 to the length L4 of the excretion-section slit region S in the lateral direction Y is preferably 1 or less, more preferably 5/6 or less, and preferably 2/5 or greater, more preferably 1/2 or greater.

The length L3, in the lateral direction Y, of the rear slit region formed by the rear slits 45 is preferably 20 mm or greater, more preferably 25 mm or greater, and preferably 50 mm or less, more preferably 45 mm or less. Further, the rear slit 45's length L6 (cf. Fig. 7(a)) in the longitudinal direction X is preferably 5% or greater, more preferably 7.5% or greater, and preferably 17.5% or less, more preferably 15% or less, of the length L3 in the lateral direction Y.

As illustrated in Figs. 1(a) and 2, the absorbent member 4 of the napkin 1 of the present embodiment includes, in the excretion-section opposing region B, the multi-layer section 42 in which the number of layers of absorbent sheets is larger than that in sections located in its periphery, and the central slit region S1 is formed in the multi-layer section 42. On the other hand, the rear slits 45 are formed in a section that is located in the rear of the multi-layer section 42 and that has a smaller number of layers of absorbent sheets than the multi-layer section 42. Thus, in the central slit region S1 formed in the excretion-section opposing region B, because pressure from the thighs is applied and the body's shape and movement are complicated, rigidity is increased by increasing the layers, and thus twisting can be prevented. On the other hand, because the rear slit region is formed in a section that contacts the buttocks, deformation is not as complicated as in the central region. Further, it is possible to prevent uncomfortableness particularly when the wearer is seated.

In cases where the process for manufacturing the napkin 1 involves a step of transporting the absorbent member, or a continuous absorbent member, along its length direction and meanwhile forming rear slits that extend along a direction orthogonal to the transporting direction, the absorbent member etc. after the formation of the slits may be pulled in the transporting direction and fixed between e.g. upper and lower sheets in this state; in this way, a gap 46 in which the rear slit 45 is opened can be formed in the slit's central section 45a in the length direction, as illustrated in Fig. 7. In the rear slit 45 of the present invention, the central section 45a is located more toward the front region than the positions of both end sections 45b, 45b, and the central section 45a has a gap 46 formed by opening the slit. Thus, deformability into a shape that conforms to the body shape is further improved. If, however, sections other than the central section 45a are opened widely, absorption in sections overlapping the rear slits 45 may be inhibited. Thus, it is preferable that the rear slit 45 has non-opened regions in the vicinity of the respective end sections 45b in the length direction. By forming the rear slits 45 in a section which is located in the rear of the multi-layer section 42 and which has a lower rigidity than the multi-layer section, it is possible to moderately neck-in the absorbent member etc. by pulling, in the transporting direction, the absorbent member etc. after the formation of the slits, and it is also possible to easily form rear slits 45 each having a gap in the central section 45a and slit-non-opened regions in the respective end sections 45b, 45b.

From the viewpoint of improving deformability by the rear slits 45 and also preventing absorption from being inhibited by the formation of an opening over the entire length of the slit, in each rear slit 45 having a gap 46 in its central section 45a, the width L7 (cf. Fig. 7(a)) in the central section 45a is preferably 0.05% or greater, more preferably 0.075% or greater, and preferably 10% or less, more preferably 8% or less, of the length L3, in the lateral direction Y, of the rear slit region S3 formed by the rear slits 45. From the same viewpoint, the slit 45's width L7 (cf. Fig. 7(a)) in the central section 45a is preferably 0.3% or greater, more preferably 0.5% or greater, and preferably 50% or less, more preferably 35% or less, of the rear slit 45's length L6 in the longitudinal direction X. From the viewpoint of preventing the inhibition of absorption, it is preferable that the gap 46 in the central section 45a of the rear slit 45 is of a width that disappears when the absorbent member 4 is curved along the body shape during use of the napkin 1.

Further, as illustrated in Fig. 1(b), the rear slits 45 in the absorbent member 4 of the present embodiment are formed in such a manner that, among two rear slits adjacent to one another in the longitudinal direction X of the napkin, the rear end position PI of the rear slit located in the front is located more toward the front than the front end position P2 of the rear slit located in the rear. By arranging the rear slits 45 in this way, a slit-less region can be formed between adjacent rear slits 45 in the longitudinal direction X. Thus, at the time of forming a slit in the absorbent member during product processing, pressure from a slitting blade can be prevented from being dispersed in the width direction of the absorbent member, and a slit can be formed neatly from the apex to both end sections. Further, when the rear slits are attached along the buttocks, the absorbent member is curved so that either the opened apex closes or the opposing sides of the opening slightly overlap; this structure is also advantageous in terms of easy deformability so as to conform to the body shape. From this viewpoint, the distance L8 (Fig. 1(b)) between the rear end position PI of the front-side rear slit and the front end position P2 of the adjacent slit on the rear side in the longitudinal direction X is preferably 2 mm or greater, more preferably 4 mm or greater. From the viewpoint of fittability to the buttocks, the distance L8 is preferably 10 mm or less, more preferably 7 mm or less. From the same viewpoint, the distance L8 is preferably 3% or greater, more preferably 5% or greater, and preferably 30% or less, more preferably 40% or less, of the length L3 of the rear slit region S3 formed by the rear slits 45. It should be noted that, as illustrated in Fig. 1(b), the absorbent member 4 in the present embodiment has, in the absorbent member 4's peripheral edge section, an annular non-slit region in which there are no slits.

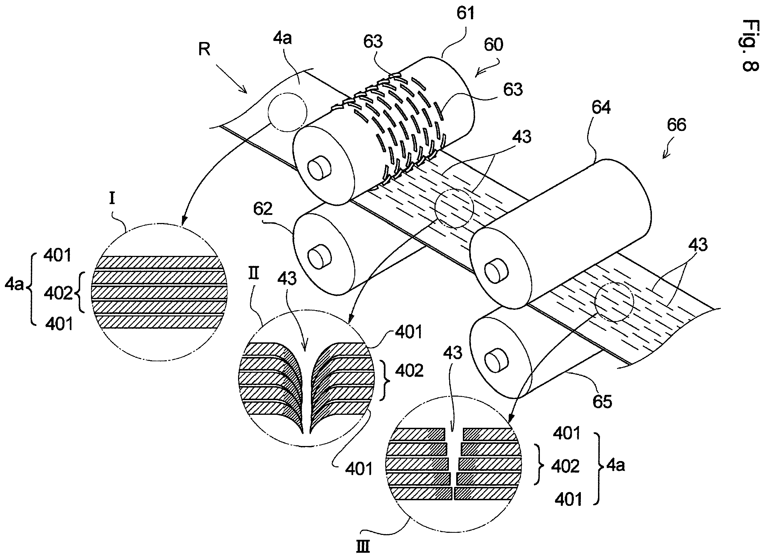

Fig. 8 schematically illustrates a process for manufacturing an absorbent member 4 of the present embodiment. This process for manufacturing an absorbent member 4 employs a cutting device 60 including: a cutter roller 61 that has a multitude of cutting blades 63 formed on its peripheral surface in a manner dispersed in the roller's circumferential direction and axial direction; and an anvil roller 62 that receives the cutting blades 63 of the cutter roller 61. This process also uses a nipping device 66 including a pair of nip rollers 64, 65 each having a smooth surface.

In the cutting device 60, the cutter roller 61 and the anvil roller 62 are arranged in such a manner that their axial centers are parallel to one another. A clearance may be provided between the cutter roller 61 and the anvil roller 62, or the two rollers may be in contact with one another. The cutting blades 63 located on the peripheral surface of the cutter roller 61 are formed and arranged so as to match the shapes of the slits 43, 45 to be formed in the target absorbent member 4. It should be noted that Fig. 8 illustrates only the cutting blades 63 for forming the longitudinal slits 43. In the nipping device 66, the pair of nip rollers 64, 65 is arranged in such a manner that their axial centers are parallel to one another. A clearance may be provided between the pair of nip rollers 64, 65, or the two rollers may be in contact with one another. The axial centers of the rollers 61, 62 of the cutting device 60 are parallel to the axial centers of the rollers 64, 65 of the nipping device 66.

An original textile 4a of the absorbent member 4, which has a layered structure including the base absorbent sheet and the central absorbent sheet, is transported in the direction indicated by arrow R in Fig. 8, and is supplied between the cutter roller 61 and the anvil roller 62 in the cutting device 60. The state of the thickness-wise cross section of the original textile 4a before being supplied between the rollers 61, 62 is illustrated in the circle indicated by I in Fig. 8. In the original textile 4a before being supplied between the rollers 61, 62, the absorbent sheets 401, 402 are in a non-joined state.

When the original textile 4a is supplied between the rollers 61, 62, the cutting blades 63, which are provided on the peripheral surface of the cutter roller 61, advance into the original textile 4a along the thickness direction of the original textile 4a by compression from the rollers 61, 62. Thus, longitudinal slits 43 and rear slits (not illustrated) are formed in the original textile 4a. The state of the thickness-wise cross section of the original textile 4a immediately after a longitudinal slit 43 has been formed is illustrated in the circle indicated by II in Fig. 8. Immediately after the formation of a longitudinal slit 43, in a section in the vicinity of where the longitudinal slit 43 has been formed, the absorbent sheets 401, 402 are in a state protruding from one surface toward the other surface because of the advancement of the cutting blade 63 into the original textile 4a. The "one surface" is the surface in contact with the cutter roller 61, and the "other surface" is the surface in contact with the anvil roller 62.

In addition to this protruding state, in a section in the vicinity of where the longitudinal slit 43 has been formed, the protruding absorbent sheets 401, 402 are densified by the advancement of the cutting blade 63 and are in a joined state. This densification increases the density of the absorbent sheets 401, 402 in the vicinity of the longitudinal slit 43. In sections at a distance from the longitudinal slit 43, the absorbent sheets 401, 402 are not densified, or the degree of densification is small; thus, the density is not increased, or the degree of increase in density is small.

The original textile 4a, in which slits have been formed as above, is then supplied between the pair of nip rollers 64, 65 in the nipping device 66. The state of the thickness-wise cross section of the original textile 4a after being nipped by the rollers 64, 65 is illustrated in the circle indicated by III in Fig. 8. By being nipped by the rollers 64, 65, the protruding section--which consists of the absorbent sheets 401, 402 and has been created in the vicinity of the longitudinal slit 43--is pressed back into the longitudinal slit 43, and the absorbent sheets 401, 402 are flattened. As the protruding section is pressed back, the absorbent sheets 401, 402, which have been joined together up to this point, are separated, and voids are created between the absorbent sheets 401, 402. Thus, the absorbent sheets 401, 402 return to their non-joined state, which is the state before the formation of the slits.

When the cutting blades 63 provided on the cutter roller 61 of the cutting device 60 advance into the original textile 4a in the thickness direction, the closer the absorbent sheet is located to the cutter roller 61, the greater the extent of cutting by each cutting blade 63; conversely, the farther the absorbent sheet is located from the cutter roller 61, the smaller the extent of cutting. As a result, in the original textile 4a that has been flattened by being nipped by the nipping device 66, the closer the absorbent sheet is located to the cutter roller 61, the wider the slit width; conversely, the farther the absorbent sheet is located from the cutter roller 61, the narrower the slit width. The difference between the wide part and the narrow part in the slit width becomes more significant as the thickness of the original textile 4a increases or as the number of layers of the absorbent sheets 401, 402 increases.

In this way, slits (longitudinal slits 43 and rear slits 45) having the intended shapes can be formed successfully.

Materials for forming the various members of the napkin 1 are described below. As for the topsheet 2 and the backsheet 3, it is possible to use various materials conventionally used in this technical field without particular limitation. For example, for the topsheet 2, it is possible to use a liquid-permeable sheet, such as one of various nonwoven fabrics subjected to a hydrophilizing treatment, or a porous film. For the backsheet 3, it is possible to use a liquid-impermeable or water-repellent material. Examples of liquid-impermeable materials that may be used include: resin films; microporous moisture-permeable resin films; and laminate materials of resin films and nonwoven fabrics. Examples of water-repellent materials that may be used include: multilayer composite nonwoven fabrics, such as spun-bonded/melt-blown/spun-bonded nonwoven fabrics; spun-bonded nonwoven fabrics; heat-bonded nonwoven fabrics; and air-through nonwoven fabrics.

As for the absorbent sheets 401, 402 constituting the absorbent member 4, it is possible to preferably use a material made into a sheet by binding together constituent fibers such as pulp, and also binding the constituent fibers and an absorbent polymer together, by means of a separately added binder, such as an adhesive or an adhesive fiber, or the absorbent polymer's adhesive force that arises when the polymer is in a wet state. Other examples of absorbent sheets that may be used include: pulp-containing absorbent sheets manufactured according to the method disclosed in JP H8-246395A; dry sheets made by depositing pulverized pulp and an absorbent polymer that have been supplied on an air flow, and then binding the pulp and the polymer with an adhesive (such as a vinyl acetate adhesive or PVA); absorbent sheets obtained by applying e.g. a hot-melt adhesive between sheets of pulp-containing paper or nonwoven fabric, and then sprinkling absorbent polymer particles thereon; and absorbent sheets obtained by blending a superabsorbent polymer during a step for manufacturing a spun-bonded or melt-blown nonwoven fabric. Such absorbent sheets can be used as a sheet-like absorbent member by cutting a single sheet into a predetermined shape. The thickness of a single absorbent sheet is preferably 0.1 mm or greater, more preferably 0.3 mm or greater, and 2 mm or less, more preferably 1.5 mm or less, and more specifically, preferably from 0.1 mm to 2 mm inclusive, more preferably from 0.3 mm to 1.5 mm inclusive.

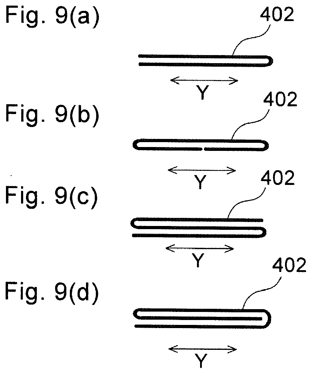

In the present embodiment, each of the base absorbent sheet 401 and the central absorbent sheet 402 constituting the absorbent member 4 is folded up to form a layered structure including a plurality of layers. Instead, a plurality of individual absorbent sheets may be layered. Further, the absorbent sheet may be folded up according to various folding methods. For example, the auxiliary absorbent member 41 in the present embodiment may be obtained by folding the central absorbent sheet 402 according to the various folding methods illustrated in Figs. 9(a) to 9(d).

The thickness of the multi-layer section 42 in the absorbent member 4 is preferably 0.7 mm or greater, more preferably 1 mm or greater, and preferably 5 mm or less, more preferably 4 mm or less, and more specifically, preferably from 0.7 mm to 5 mm inclusive, more preferably from 1 mm to 4 mm inclusive. By setting the thickness of the multi-layer section 42 within the aforementioned range, it is possible to easily achieve both high absorption performance and an excellent feel in the excretion-section opposing region B, which is where the multi-layer section 42 is formed, when the diaper is worn. Further, in cases where the absorbent article includes wings 7 as in the napkin 1 of the present embodiment, twisting of the absorbent member 4 in the excretion-section opposing region B can be suppressed more easily when the absorbent article is worn. Further, the thickness in sections of the absorbent member 4 other than the multi-layer section 42 is preferably 0.3 mm or greater, more preferably 0.5 mm or greater, and preferably 3 mm or less, more preferably 2.5 mm or less, and more specifically, preferably from 0.3 mm to 3 mm inclusive, more preferably from 0.5 mm to 2.5 mm inclusive. Setting the thickness within the aforementioned range is preferable from the viewpoint of achieving high absorption performance and improving followability to the wearer's movement.

In the absorbent member 4, the difference in thickness (i.e., the step) at the boundary between the multi-layer section 42 and the peripheral section or in the boundary's vicinity is preferably 2 mm or less, more preferably 1.5 mm or less. The aim of this range is to suppress, in the excretion-section opposing region B, the occurrence of twisting in the vicinity of the boundary between the multi-layer section 42 and a region in its peripheral section where only the main absorbent member 40 is present. If the step is too large, when the napkin 1 follows the wearer's movement while it is worn, the napkin attempts to fill in the gap created by the step, and this may result in that the napkin is prone to getting twisted because of the presence of the step. In contrast, by setting the step within the aforementioned range, the effect of suppressing the occurrence of twisting in the excretion-section opposing region B is enhanced. The thickness of each of the members of the absorbent member 4 is measured according to the following method.

### Method for Measuring Thickness of Absorbent Member:

An absorbent member, which is the object to be measured, is placed on a horizontal place in such a manner that there are no creases or folds/bends, and the thickness is measured under a load of 5 cN/cm². In the thickness measurement of the present invention, a thickness gauge (Peacock Dial Upright Gauge R5-C from Ozaki Mfg. Co., Ltd.) is used. At the time of measurement, a plate (an approx. 5-mm-thick acrylic plate) having a circular or square shape in a planar view is arranged between the tip of the thickness gauge and a section to be measured in the object; the size of the plate is adjusted so that the load is 5 cN/cm².

Examples of absorbent polymers included in the absorbent sheets 401, 402 include sodium polyacrylate, (acrylic acid-vinyl alcohol) copolymers, crosslinked sodium polyacrylate, (starch-acrylic acid) graft copolymers, (isobutylene-maleic anhydride) copolymers and saponification products thereof, and polyaspartic acid. It is preferable that both the absorbent sheets 401, 402 constituting the absorbent member 4 are absorbent sheets including an absorbent polymer, but an absorbent sheet including an absorbent polymer and an absorbent sheet not including an absorbent polymer may be used in combination as a laminate of absorbent sheets constituting the absorbent member.

For the side sheets 10, it is possible to use various materials conventionally used in this technical field without particular limitation; for example, a liquid-impermeable or water-repellent resin film or a laminate of a resin film and a nonwoven fabric may be used. Examples of other materials include water-repellent (hydrophobic) nonwoven fabrics, such as spun-bonded nonwoven fabrics, sheets in which spun-bonded nonwoven fabrics (S) and melt-blown nonwoven fabrics (M) have been composited (e.g. SM, SMS, and SMMS), heat-rolled nonwoven fabrics, and air-through nonwoven fabrics. Particularly, the use of a water-repellent air-through nonwoven fabric is preferred from the viewpoint of pleasant texture and side leakage prevention.

The present invention has been described above according to preferred embodiments thereof, but the present invention is not limited to the foregoing embodiments. For example, the napkin 1 does not have to include wings 7. Further, the napkin may include rear flaps which are provided on respective sides of the absorbent assembly 5 in the rear region and which are spread out and fixed to the inner surface of underpants. The excretion-section slit region S may be formed only in the excretion-section opposing region B, or may extend from the excretion-section opposing region B up to a portion of the rear region C, or may extend from the excretion-section opposing region B up to a portion of the front region A and a portion of the rear region C. Further, the number of layers of absorbent sheets in the multi-layer section 42 is not limited to the number of layers illustrated in Fig. 2. Also, the difference in the number of layers of absorbent sheets between the multi-layer section 42 and sections other than the multi-layer section is not limited to the number illustrated in Fig. 2.

Further, there may be two, three, or five or more rear slits 45. Also, rear slits with a gap 46 opened in the central section 45a and rear slits without such a gap may co-exist. The rear slit 45 may have a shape formed by linking two straight lines at an angle in the slit's central section. Further, although it is preferable that the aforementioned slits penetrate the absorbent member 4 as described above, the slits do not have to penetrate the absorbent member; alternatively, only a portion of the slits may be non-penetrating. Examples of non-penetrating slits include slits that are not opened on the skin-opposing surface side or the non-skin-opposing surface side of the absorbent member 4.

Further, although it is preferable to provide no space between two slit rows R1, R2 adjacent to one another in the longitudinal direction X as described above, a space may be provided between the slit rows R1, R2 to the extent that the effects achieved by the present invention are not impaired.

In addition to sanitary napkins, the absorbent article of the present invention is applicable to panty liners (vaginal discharge sheets), incontinence pads, and the like.

In relation to the foregoing embodiments of the invention, the following additional features (absorbent articles, etc.) are further disclosed.
{1} An absorbent article comprising an absorbent assembly that includes: a liquid-retentive absorbent member; a topsheet arranged on a skin-opposing surface side of the absorbent member; and a backsheet arranged on a non-skin-opposing surface side of the absorbent member, wherein:
   the absorbent assembly has: an excretion-section opposing region arranged so as to oppose an excretion section of a wearer when the absorbent article is worn; a front region arranged more toward the wearer's front side than the excretion-section opposing region; and a rear region arranged more toward the wearer's rear side than the excretion-section opposing region;
   the absorbent article has a longitudinal direction corresponding to the wearer's front/rear direction, and a lateral direction orthogonal to the longitudinal direction;
   the absorbent member has a layered structure which has absorbent sheets including pulp and which has a plurality of layers;
   the layered structure of the absorbent sheets includes: a central absorbent sheet located in the excretion-section opposing region; and a base absorbent sheet that is layered on the central absorbent sheet so as to extend outward from at least a portion of a peripheral edge of the central absorbent sheet;
   a plurality of longitudinal slits are formed in a dispersed state in the excretion-section opposing region of the absorbent member, each longitudinal slit being cut toward a thickness direction of the absorbent member and along the longitudinal direction; and
   in the layered structure, at least in the excretion-section opposing region, a portion of the plurality of layers of the absorbent sheets forming the layered structure is in a non-joined state in a section where each longitudinal slit is formed.
{2} The absorbent article as set forth in clause {1}, wherein, when the longitudinal slit's length in the lateral direction is viewed along the thickness direction of the absorbent member, the slit's length W1 on the topsheet side is longer than the slit's length W2 on the backsheet side.
{3} The absorbent article as set forth in clause {2}, wherein the ratio W1/W2 between the length W1 on the topsheet side and the length W2 on the backsheet side is from 5 to 80 inclusive.
{4} The absorbent article as set forth in any one of clauses {1} to {3}, wherein a density of the absorbent sheets in each longitudinal slit's vicinity is higher than a density of the absorbent sheets in a non-slit section located between the longitudinal slits.
{5} The absorbent article as set forth in clause {4}, wherein the density of the absorbent sheets in each longitudinal slit's vicinity is from 1.05 times to twice the density of the absorbent sheets in the non-slit section.
{6} The absorbent article as set forth in any one of clauses {1} to {5}, wherein:
   a plurality of slit rows are formed in the longitudinal direction, each slit row including a plurality of the longitudinal slits separated from one another in the lateral direction;
   there is no space between the slit rows adjacent to one another in the longitudinal direction; and
   the positions of the slits in the slit rows adjacent to one another in the longitudinal direction are misaligned in the lateral direction.
{7} The absorbent article as set forth in any one of clauses {1} to {6}, wherein:
   a length of each longitudinal slit along the longitudinal direction is from 10 mm to 35 mm inclusive;
   a distance between the longitudinal slits in each slit row, which includes a plurality of the longitudinal slits separated from one another in the lateral direction, is from 3 mm to 20 mm inclusive; and
   an area from each end section of the absorbent member to a point that is from 5 mm to 15 mm inward from the end section constitutes a non-slit section.
{8} The absorbent article as set forth in any one of clauses {1} to {7}, wherein:
   the absorbent member includes, in the rear region, rear slits that extend along a direction intersecting with the longitudinal direction;
   each rear slit has a planar shape in which the slit's central section in its length direction is located more toward the front region than positions of both end sections of the slit in its length direction; and
   the plurality of rear slits are formed with an interval therebetween in the longitudinal direction.
{9} The absorbent article as set forth in clause {8}, wherein, in the layered structure, a portion of the plurality of layers of the absorbent sheets forming the layered structure is in a non-joined state in the rear region.
{10} The absorbent article as set forth in clause {8} or {9}, wherein the rear slit has a shape, in a planar view, that is curved protrudingly toward the front region.
{11} The absorbent article as set forth in any one of clauses {8} to {10}, wherein a length, in the lateral direction, of a rear slit region in which the rear slits are arranged is equal to or shorter than a length, in the lateral direction, of an excretion-section slit region in which the longitudinal slits are arranged.
{12} The absorbent article as set forth in any one of clauses {1} to {11}, wherein:
   the excretion-section slit region in which the longitudinal slits are arranged includes a central slit region located in a central section of the absorbent member in the lateral direction, and side slit regions each located outward, in the lateral direction, of the central slit region; and
   in the central slit region, the longitudinal slits penetrate the layered structure including the central absorbent sheet and the base absorbent sheet.
{13} The absorbent article as set forth in clause {12}, wherein, in the side slit region, the longitudinal slits are formed so as to penetrate the layered structure including the base absorbent sheet.
{14} The absorbent article as set forth in clause {12} or {13}, wherein the section, in which each longitudinal slit is formed, of the side slit region has the layered structure of the absorbent sheets which is in a non-joined state.
{15} The absorbent article as set forth in any one of clauses {1} to {14}, wherein the central absorbent sheet is arranged inside the base absorbent sheet which is in a state where both ends thereof are folded up.
{16} The absorbent article as set forth in any one of clauses {1} to {15}, wherein the longitudinal slits are not formed in the topsheet which is arranged on an upper side of the absorbent member and the backsheet which is arranged on a lower side of the absorbent member.
{17} The absorbent article as set forth in any one of clauses {1} to {16}, wherein the absorbent sheet is made into a sheet by binding constituent fibers together, and also binding the constituent fibers and an absorbent polymer together, by means of a separately added binder or an adhesive force of the absorbent polymer.
{18} The absorbent article as set forth in any one of clauses {1} to {17}, wherein each of the base absorbent sheet and the central absorbent sheet is folded up to form a layered structure including a plurality of layers.
{19} The absorbent article as set forth in any one of clauses {1} to {18}, wherein:
   a section where the central absorbent sheet is arranged is a multi-layer section having a thickness that is thicker than that of peripheral sections; and
   the thickness of the multi-layer section is from 0.7 mm to 5 mm inclusive.
{20} The absorbent article as set forth in clause {19}, wherein a thickness in sections other than the multi-layer section is from 0.3 mm to 3 mm inclusive.
{21} The absorbent article as set forth in clause {19} or {20}, wherein a difference in thickness at a boundary between the multi-layer section and the peripheral section or in the boundary's vicinity is 2 mm or less.
{22} The absorbent article as set forth in any one of clauses {1} to {21}, wherein:
   a second sheet is provided between the absorbent member and the topsheet; and
   the topsheet and the second sheet are partially fixed.
{23} The absorbent article as set forth in any one of clauses {1} to {22}, wherein the absorbent article comprises:
   the absorbent assembly including the absorbent member, the topsheet, and the backsheet; and
   a pair of wings extending outwardly in the lateral direction from the absorbent assembly's respective lateral sides that extend along the longitudinal direction.
{24} The absorbent article as set forth in any one of clauses {1} to {23}, wherein the absorbent article is a sanitary napkin.
{25} A method for manufacturing the absorbent article as set forth in clause {1}, wherein the longitudinal slits are formed by:
   a step in which an absorbent-member original textile having a layered structure including a base absorbent sheet and a central absorbent sheet is supplied between a cutter roller that has cutting blades on its peripheral surface and an anvil roller that opposes the cutter roller, and slits are formed in the original textile; and
   a step in which the original textile having the slits formed therein is supplied between a pair of rollers.

### Industrial Applicability

The absorbent article of the present invention can instantaneously absorb excreted fluid into the absorbent member, can easily deform in response to external force in a state where it is worn, and is less likely to cause uncomfortableness when worn.

## Claims

1. An absorbent article comprising an absorbent assembly that includes: a liquid-retentive absorbent member; a topsheet arranged on a skin-opposing surface side of the absorbent member; and a backsheet arranged on a non-skin-opposing surface side of the absorbent member, wherein:
the absorbent assembly has: an excretion-section opposing region arranged so as to oppose an excretion section of a wearer when the absorbent article is worn; a front region arranged more toward the wearer's front side than the excretion-section opposing region; and a rear region arranged more toward the wearer's rear side than the excretion-section opposing region;
the absorbent article has a longitudinal direction corresponding to the wearer's front/rear direction, and a lateral direction orthogonal to the longitudinal direction;
the absorbent member has a layered structure which has absorbent sheets including pulp and which has a plurality of layers;
the layered structure of the absorbent sheets includes: a central absorbent sheet located in the excretion-section opposing region; and a base absorbent sheet that is layered on the central absorbent sheet so as to extend outward from at least a portion of a peripheral edge of the central absorbent sheet;
a plurality of longitudinal slits are formed in a dispersed state in the excretion-section opposing region of the absorbent member, each longitudinal slit being cut toward a thickness direction of the absorbent member and along the longitudinal direction; and
in the layered structure, at least in the excretion-section opposing region, a portion of the plurality of layers of the absorbent sheets forming the layered structure is in a non-joined state in a section where each longitudinal slit is formed,
an excretion-section slit region in which the longitudinal slits are arranged includes: a central slit region located in a central section of the absorbent member in the lateral direction; and side slit regions each located outward, in the lateral direction, of the central slit region; and
in the central slit region, the longitudinal slits penetrate the layered structure including the central absorbent sheet and the base absorbent sheet.

2. The absorbent article according to claim 1, wherein a density of the absorbent sheets in each longitudinal slit's vicinity is higher than a density of the absorbent sheets in a non-slit section located between the longitudinal slits.

3. The absorbent article according to claim 1 or 2, wherein:
a plurality of slit rows are formed in the longitudinal direction, each slit row including a plurality of the longitudinal slits separated from one another in the lateral direction;
there is no space between the slit rows adjacent to one another in the longitudinal direction; and
the positions of the slits in the slit rows adjacent to one another in the longitudinal direction are misaligned in the lateral direction.

4. The absorbent article according to any one of claims 1 to 3, wherein, in the side slit region, the longitudinal slits are formed so as to penetrate the layered structure including the base absorbent sheet.

5. The absorbent article according to any one of claims 1 to 4, wherein the section, in which each longitudinal slit is formed, of the side slit region has the layered structure of the absorbent sheets which is in a non-joined state.

6. The absorbent article according to any one of claims 1 to 5, wherein the central absorbent sheet is arranged inside the base absorbent sheet which is in a state where both ends thereof are folded up.

7. The absorbent article according to any one of claims 1 to 6, wherein the absorbent sheet is made into a sheet by binding constituent fibers together, and also binding the constituent fibers and an absorbent polymer together, by means of a separately added binder or an adhesive force of the absorbent polymer.

8. The absorbent article according to any one of claims 1 to 7, wherein each of the base absorbent sheet and the central absorbent sheet is folded up to form a layered structure including a plurality of layers.

9. The absorbent article according to any one of claims 1 to 8, wherein:
a second sheet is further provided between the absorbent member and the topsheet; and
the topsheet and the second sheet are partially fixed.

10. The absorbent article according to any one of claims 1 to 9, wherein, when the longitudinal slit's length in the lateral direction is viewed along the thickness direction of the absorbent member, the slit's length W1 on the topsheet side is longer than the slit's length W2 on the backsheet side.

11. The absorbent article according to any one of claims 1 to 10, wherein:
the absorbent member includes, in the rear region, rear slits that extend along a direction intersecting with the longitudinal direction;
each rear slit has a planar shape in which the slit's central section in its length direction is located more toward the front region than positions of both end sections of the slit in its length direction; and
the plurality of rear slits are formed with an interval therebetween in the longitudinal direction.

12. The absorbent article according to claim 11, wherein, in the layered structure, a portion of the plurality of layers of the absorbent sheets forming the layered structure is in a non-joined state in the rear region.

13. A method for manufacturing the absorbent article according to claim 1, wherein the longitudinal slits are formed by:
a step in which an absorbent-member original textile having a layered structure including a base absorbent sheet and a central absorbent sheet is supplied between a cutter roller that has cutting blades on its peripheral surface and an anvil roller that opposes the cutter roller, and slits are formed in the original textile; and
a step in which the original textile having the slits formed therein is supplied between a pair of rollers.

## Patentansprüche

1. Saugfähiger Artikel, der eine saugfähige Anordnung aufweist, die umfasst: ein flüssigkeitsrückhaltendes saugfähiges Element; eine Decklage, die auf einer der Haut gegenüberliegenden Oberflächenseite des saugfähigen Elements angeordnet ist; und eine Rückenlage, die auf einer der Haut nicht gegenüberliegenden Oberflächenseite des saugfähigen Elements angeordnet ist, wobei:
die saugfähige Anordnung aufweist: einen einem Ausscheidungsabschnitt gegenüberliegenden Bereich, der so angeordnet ist, dass er einem Ausscheidungsabschnitt eines Trägers gegenüberliegt, wenn der saugfähige Artikel getragen wird; einen vorderen Bereich, der mehr zur Vorderseite des Trägers angeordnet ist als der einem Ausscheidungsabschnitt gegenüberliegende Bereich; und einen hinteren Bereich, der mehr zur Rückseite des Trägers angeordnet ist als der einem Ausscheidungsabschnitt gegenüberliegende Bereich;
der saugfähige Artikel eine Längsrichtung, die der Vorwärts-/Rückwärtsrichtung des Trägers entspricht, und eine zur Längsrichtung orthogonale laterale Richtung aufweist;
das saugfähige Element einen Schichtaufbau aufweist, der saugfähige Lagen aufweist, die Zellstoff enthalten, und der eine Vielzahl von Schichten aufweist;
der Schichtaufbau der saugfähigen Lagen umfasst: eine mittlere saugfähige Lage, die in dem einem Ausscheidungsabschnitt gegenüberliegenden Bereich angeordnet ist; und eine saugfähige Basislage, die auf die mittlere saugfähige Lage geschichtet ist, so dass sie sich von mindestens einem Abschnitt einer Umfangskante der mittleren saugfähigen Lage nach außen erstreckt;
eine Vielzahl von longitudinalen Schlitzen in einem verteilten Zustand in dem einem Ausscheidungsabschnitt gegenüberliegenden Bereich des saugfähigen Elements ausgebildet sind, wobei jeder longitudinale Schlitz in eine Dickenrichtung des saugfähigen Elements und längs der Längsrichtung geschnitten ist; und
im Schichtaufbau mindestens in dem einem Ausscheidungsabschnitt gegenüberliegenden Bereich ein Anteil der Vielzahl der Schichten der saugfähigen Lagen, die den Schichtaufbau bilden, in einem Abschnitt, wo jeder longitudinale Schlitz ausgebildet ist, sich in einem nicht verbundenen Zustand befindet,
ein Ausscheidungsabschnitt-Schlitzbereich, in dem die longitudinalen Schlitze angeordnet sind, umfasst: einen mittleren Schlitzbereich, der in einem mittleren Abschnitt des saugfähigen Elements in die laterale Richtung angeordnet ist; und seitliche Schlitzbereiche, die jeweils in die laterale Richtung des mittleren Schlitzbereichs nach außen angeordnet sind; und
im mittleren Schlitzbereich die longitudinalen Schlitze den Schichtaufbau einschließlich der mittleren saugfähigen Lage und der saugfähigen Basislage durchdringen.

2. Saugfähiger Artikel nach Anspruch 1, wobei eine Dichte der saugfähigen Lagen in der Nähe jedes longitudinalen Schlitzes höher ist als eine Dichte der saugfähigen Lagen in einem schlitzfreien Abschnitt, der zwischen den longitudinalen Schlitzen angeordnet ist.

3. Saugfähiger Artikel nach Anspruch 1 oder 2, wobei:
eine Vielzahl von Schlitzreihen in die Längsrichtung ausgebildet ist, wobei jede Schlitzreihe eine Vielzahl der longitudinalen Schlitze umfasst, die in die laterale Richtung voneinander getrennt sind;
es keinen Platz zwischen den in die Längsrichtung zueinander benachbarten Schlitzreihen gibt; und
die Positionen der Schlitze in den in die Längsrichtung zueinander benachbarten Schlitzreihen in die laterale Richtung nicht fluchten.

4. Saugfähiger Artikel nach einem der Ansprüche 1 bis 3, wobei die longitudinalen Schlitze im seitlichen Schlitzbereich so ausgebildet sind, dass sie den Schichtaufbau einschließlich der saugfähigen Basislage durchdringen.

5. Saugfähiger Artikel nach einem der Ansprüche 1 bis 4, wobei der Abschnitt des seitlichen Schlitzbereichs, in dem jeder longitudinale Schlitz ausgebildet ist, den Schichtaufbau der saugfähigen Lagen aufweist, die sich in einem nicht verbundenen Zustand befinden.

6. Saugfähiger Artikel nach einem der Ansprüche 1 bis 5, wobei die mittlere saugfähige Lage innerhalb der saugfähigen Basislage angeordnet ist, die sich in einem Zustand befindet, in dem deren beide Enden zusammengelegt sind.

7. Saugfähiger Artikel nach einem der Ansprüche 1 bis 6, wobei die saugfähige Lage durch Binden von Komponentenfasern aneinander und außerdem durch Binden der Komponentenfasern und eines saugfähigen Polymers aneinander mittels eines getrennt hinzugefügten Bindemittels oder einer Adhäsionskraft des saugfähigen Polymers zu einer Lage verarbeitet wird.

8. Saugfähiger Artikel nach einem der Ansprüche 1 bis 7, wobei jeweils die saugfähige Basislage und die mittlere saugfähige Lage zusammengelegt sind, um einen Schichtaufbau zu bilden, der eine Vielzahl von Schichten umfasst.

9. Saugfähiger Artikel nach einem der Ansprüche 1 bis 8, wobei:
ferner eine zweite Lage zwischen dem saugfähigen Element und der Decklage vorgesehen ist; und
die Decklage und die zweite Lage teilweise fixiert sind.

10. Saugfähiger Artikel nach einem der Ansprüche 1 bis 9, wobei, wenn die Länge des longitudinalen Schlitzes in die laterale Richtung längs der Dickenrichtung des saugfähigen Elements betrachtet wird, die Länge W1 des Schlitzes auf der Decklagenseite länger als die Länge W2 des Schlitzes auf der Rückenlagenseite ist.

11. Saugfähiger Artikel nach einem der Ansprüche 1 bis 10, wobei:
das saugfähige Element im hinteren Bereich hintere Schlitze aufweist, die sich längs einer Richtung erstrecken, die sich mit der Längsrichtung schneidet;
jeder hintere Schlitz eine ebene Form aufweist, in der der mittlere Abschnitt des Schlitzes in seiner Längenrichtung mehr zum vorderen Bereich angeordnet ist als Positionen beider Endabschnitte des Schlitzes in seine Längenrichtung; und
die Vielzahl der hinteren Schlitze mit einem Intervall dazwischen in die Längsrichtung ausgebildet sind.

12. Saugfähiger Artikel nach Anspruch 11, wobei im Schichtaufbau ein Abschnitt der Vielzahl der Schichten der saugfähigen Lagen, die den Schichtaufbau bilden, sich im hinteren Bereich in einem nicht verbundenen Zustand befindet.

13. Verfahren zum Herstellen des saugfähigen Artikels nach Anspruch 1, wobei die longitudinalen Schlitze gebildet werden durch:
einen Schritt, in dem ein Ausgangsgewebe des saugfähigen Elements, das einen Schichtaufbau aufweist, der eine saugfähige Basislage und eine mittlere saugfähige Lage umfasst, zwischen eine Schneidwalze, die auf ihrer Umfangsfläche Schneidmesser aufweist, und eine Ambosswalze zugeführt wird, die der Schneidwalze gegenüberliegt, und Schlitze im Ausgangsgewebe gebildet werden; und
einen Schritt, in dem das Ausgangsgewebe, das die darin ausgebildeten Schlitze aufweist, zwischen ein Walzenpaar zugeführt wird.

## Revendications

1. Article absorbant comprenant un ensemble absorbant comportant : un élément absorbant rétenteur de liquide ; une feuille supérieure disposée sur un côté de la surface opposé à la peau de l'élément absorbant ; et une feuille arrière disposée sur un côté de la surface non opposé à la peau de l'élément absorbant, où :
l'ensemble absorbant présente : une zone opposée à la section d'excrétion disposée de manière à être opposée à une section d'excrétion d'un porteur quand l'article absorbant est porté ; une zone avant disposée plus vers le côté avant du porteur que la zone opposée à la section d'excrétion ; et une zone arrière disposée plus vers le côté arrière du porteur que la zone opposée à la section d'excrétion ;
ledit article absorbant présente une direction longitudinale correspondant à la direction du côté avant vers le côté arrière du porteur, et une direction latérale orthogonale à la direction longitudinale ;
l'élément absorbant a une structure stratifiée comprenant des feuilles absorbantes contenant de la pâte à papier et avec une pluralité de couches ;
la structure stratifiée des feuilles absorbantes présente : une feuille absorbante centrale située dans la zone opposée à la section d'excrétion ; et une feuille absorbante de base couchée sur la feuille absorbante centrale de manière à s'étendre vers l'extérieur depuis au moins une partie d'un bord périphérique de la feuille absorbante centrale ;
une pluralité de fentes longitudinales sont formées en état de dispersion dans la zone opposée à la section d'excrétion de l'élément absorbant, chaque fente longitudinale étant découpée dans le sens de l'épaisseur de l'élément absorbant et dans la direction longitudinale ; et où,
dans la structure stratifiée, au moins dans la zone opposée à la section d'excrétion, une partie de la pluralité de couches des feuilles absorbantes formant la structure stratifiée est dans un état exempt de liaison dans une section où chaque fente longitudinale est formée,
une zone de fentes de section d'excrétion où les fentes longitudinales sont ménagées comprend : une zone de fentes centrale située dans une section centrale de l'élément absorbant en direction latérale ; et des zones de fentes latérales situées chacune vers l'extérieur de la zone de fentes centrale en direction latérale ; et où,
dans la zone de fentes centrale, les fentes longitudinales pénètrent dans la structure stratifiée comprenant la feuille absorbante centrale et la feuille absorbante de base.

2. Article absorbant selon la revendication 1, où la densité des feuilles absorbantes à proximité de chaque fente longitudinale est supérieure à la densité des feuilles absorbantes dans une section exempte de fentes située entre les fentes longitudinales.

3. Article absorbant selon la revendication 1 ou la revendication 2, où :
une pluralité de rangées de fentes est formée dans la direction longitudinale, chaque rangée de fentes comprenant une pluralité des fentes longitudinales séparées l'une de l'autre dans la direction latérale ;
aucun espace n'est présenté entre les rangées de fentes adjacentes l'une à l'autre dans la direction longitudinale ; et
les emplacements des fentes dans les rangées de fentes adjacentes l'une à l'autre dans la direction longitudinale sont désalignés en direction latérale.

4. Article absorbant selon l'une des revendications 1 à 3, où, dans la zone de fentes latérale, les fentes longitudinales sont formées de manière à pénétrer dans la structure stratifiée comprenant la feuille absorbante de base.

5. Article absorbant selon l'une des revendications 1 à 4, où la section de la zone de fentes latérale dans laquelle chaque fente longitudinale est formée présente la structure stratifiée des feuilles absorbantes en état exempt de liaison.

6. Article absorbant selon l'une des revendications 1 à 5, où la feuille absorbante centrale est disposée à l'intérieur de la feuille absorbante de base dans un état où les deux extrémités de celle-ci sont repliées.

7. Article absorbant selon l'une des revendications 1 à 6, où la feuille absorbante est formée comme feuille par liaison de fibres constitutives, et également par liaison de fibres constitutives avec un polymère absorbant, au moyen d'un agent liant ajouté séparément ou d'une force d'adhérence du polymère absorbant.

8. Article absorbant selon l'une des revendications 1 à 7, où la feuille absorbante de base ainsi que la feuille absorbante centrale sont repliées pour former une structure stratifiée comprenant une pluralité de couches.

9. Article absorbant selon l'une des revendications 1 à 8, où :
une deuxième feuille est en outre prévue entre l'élément absorbant et la feuille supérieure ; et où
la feuille supérieure et la deuxième feuille sont partiellement fixées.

10. Article absorbant selon l'une des revendications 1 à 9, où, la longueur de la fente longitudinale en direction latérale étant vue dans le sens de l'épaisseur de l'élément absorbant, la longueur de fente W1 sur le côté de la feuille supérieure est supérieure à la longueur de fente W2 sur le côté de la feuille arrière.

11. Article absorbant selon l'une des revendications 1 à 10, où :
l'élément absorbant présente dans la zone arrière des fentes arrières qui s'étendent dans une direction d'intersection avec la direction longitudinale ;
chaque fente arrière a une forme plane où, dans le sens de sa longueur, la section centrale de fente est située plus vers la zone avant que les emplacements des deux sections d'extrémité de la fente dans le sens de sa longueur ; et où
la pluralité de fentes arrière est formée avec un intervalle entre celles-ci dans le sens de la longueur.

12. Article absorbant selon la revendication 11, où, dans la structure stratifiée, une partie de la pluralité de couches des feuilles absorbantes formant la structure stratifiée est dans un état exempt de liaison dans la zone arrière.

13. Procédé de fabrication de l'article absorbant selon la revendication 1, où les fentes longitudinales sont formées lors :
d'une étape où un textile d'origine à élément absorbant présentant une structure stratifiée comprenant une feuille absorbante de base et une feuille absorbante centrale est amenée entre un rouleau de coupe pourvu de lames de coupe sur sa surface périphérique, et un contre-rouleau opposé au rouleau de coupe, et où des fentes sont formées dans le textile d'origine ; et lors
d'une étape où le textile d'origine où ont été formées les fentes est amené entre une paire de rouleaux.
